# EUROPEAN PATENT APPLICATION

(11) **EP 4 825 980 A1**
(43) Date of publication of application: **21.10.2026**
(21) Application number: 26172509.7
(22) Date of filing: 15.04.2026
(51) Int. Cl.: C12Q 1/6806, C12Q 1/6837, C12Q 1/6853

(54) **PRIMER COMPOSITION, KIT, AND METHOD FOR HIGH-SENSITIVITY NUCLEIC ACID DETECTION USING SYMMETRIC BARCODED PCR AMPLIFICATION AND LAMBDA EXONUCLEASE**

(30) Priority: 15.04.2025 HK 32025106066
(71) Applicant: Hai Kang Life Corporation Limited, Shatin, N.T. (HK)
(72) Inventor: YU, Cheung Hoi, Shatin, N.T., Hong Kong (HK)
(74) Representative: Cabinet Chaillot

(57) **Abstract**

The disclosure provides a primer composition, kit, and method for high-sensitivity nucleic acid detection using symmetric barcoded PCR amplification and Lambda exonuclease. The kit comprises a primer composition for symmetric PCR amplification of a target nucleic acid to produce double-stranded DNA, wherein one of the forward or reverse primer includes a universal tag sequence with a 5' phosphorylation modification. An exonuclease (such as Lambda exonuclease) selectively degrades the phosphorylated strand to yield high-purity single-stranded DNA. The single-stranded DNA is captured by immobilized capture probes on a solid support and detected by a universal detection probe containing the complementary universal tag sequence and a gold nanoparticle signal label, followed by silver staining for signal amplification. By eliminating re-annealing competition of double-stranded products, this method enables efficient multiplex detection with sensitivity as low as 10 copies/mL. It is particularly useful for point-of-care testing (POCT) applications in pathogen identification and drug-resistance gene analysis.

## Description

### CROSS-REFERENCE TO RELATED APPLICATIONS

This application claims the benefit of priority under 35 U.S.C. § 119 to Hong Kong Patent Application No. 32025106066.3, filed on April 15, 2025, the entire contents of which are incorporated herein by reference.

### STATEMENT REGARDING FEDERALLY SPONSORED RESEARCH OR DEVELOPMENT

### JOINT RESEARCH AGREEMENT

### FIELD OF THE INVENTION

The present disclosure belongs to the technical field of molecular biology detection, and specifically relates to a primer composition, a kit, and a nucleic acid detection method for nucleic acid detection.

### BACKGROUND OF THE INVENTION

This section is intended to provide background or context for the embodiments of the present disclosure. The description herein is not admitted to be prior art by virtue of its inclusion in this section.

Gene chip (DNA Microarray) technology utilizes the principle of nucleic acid molecular hybridization to perform high-throughput parallel detection of a large number of gene targets. This technology involves immobilizing probes of known sequences onto microfabricated solid-phase supports (such as glass or silicon wafers) to perform specific hybridization with labelled sample nucleic acids, achieving qualitative or quantitative analysis of nucleic acids in the sample by detecting signal intensity. However, existing hybridization-based nucleic acid detection technology systems still face significant bottlenecks.

Firstly, insufficient sensitivity is a major issue. Traditional chip hybridization typically uses double-stranded products directly amplified by symmetric PCR. After thermal denaturation, double-stranded DNA (dsDNA) is prone to rapid re-annealing, which competitively reduces the hybridization efficiency between the target single strand and the solid-phase probe, leading to false-negative results in low-copy samples.

Secondly, asymmetric PCR, as a common method for preparing single-stranded DNA (ssDNA), is inefficient and involves complex processes. This technology relies on a vast ratio of primer concentrations (e.g., 1:10-1:100), where amplification follows a linear rather than exponential growth, making it difficult to meet the requirements of trace samples. In contrast, while symmetric PCR has high amplification efficiency, the product is double-stranded and cannot be hybridized directly and efficiently.

Thirdly, in multiplex target detection, if detection probes are designed directly based on target sequences, cross-hybridization is easily triggered due to sequence homology, resulting in non-specific signals. Although next-generation sequencing (NGS) can solve some of these problems, it involves high equipment costs and long operation cycles, making it unsuitable for point-of-care testing (POCT) applications.

In summary, there is an urgent need in the art for a high-sensitivity and high-specificity nucleic acid detection solution for both single-target and multiplex-target detection scenarios. Developing corresponding novel kits and detection methods is of great value for improving the reliability of detection results.

### SUMMARY OF THE INVENTION

The object of the present disclosure is to provide a primer composition, a kit, and a nucleic acid detection method for nucleic acid detection with improved sensitivity and specificity.

To achieve the above object, the technical solution adopted by the present disclosure is as follows:
A first aspect of the present disclosure provides a kit for nucleic acid detection, comprising:
a primer composition for performing symmetric PCR amplification on a target nucleic acid to be tested to obtain a double-stranded DNA, wherein the primer composition comprises a forward primer and a reverse primer designed for the target nucleic acid to be tested, and one of the forward primer and the reverse primer comprises a universal tag sequence and contains a phosphorylation modification group at the 5' end;
an exonuclease for degrading the strand containing the 5' phosphorylation modification in the double-stranded DNA to obtain a single-stranded DNA;
a solid support immobilized with a capture probe, wherein the capture probe is capable of hybridizing with the single-stranded DNA to bind the single-stranded DNA to the solid support; and
a universal detection probe, wherein the universal detection probe comprises a universal tag sequence capable of hybridizing with the single-stranded DNA and a signal label for signal recognition.

In some embodiments, the universal tag sequence is located between the 5' phosphorylation modification group and a specific sequence.

In some embodiments, the 5' end of the other of the forward primer and the reverse primer is a hydroxyl group or another group that cannot be recognized by an exonuclease, and does not contain a universal tag sequence.

Specifically, the other of the forward primer and the reverse primer is a conventional unmodified primer.

In some embodiments, the universal tag sequence has no homology with the target nucleic acid to be tested, so as to avoid homologous hybridization with nucleic acids in a test sample.

In some embodiments, the universal tag sequence has a length of 10-30 nucleotides.

Further, the universal tag sequence has a length of 15-25 nucleotides.

Further, the universal tag sequence comprises 5'-TCT CAA CTC GTA GCT-3'.

In some embodiments, the primer composition comprises multiple sets of primer compositions for a plurality of target nucleic acids to be tested, the capture probe comprises a plurality of capture probes for the plurality of target nucleic acids to be tested, and the plurality of capture probes are respectively immobilized at different positions on the solid support.

Wherein, the universal tag sequences on the primers for different target nucleic acids to be tested and the universal tag sequences on the universal detection probes are all identical.

In some embodiments, the target nucleic acid to be tested includes, but is not limited to, pathogens, drug-resistance genes, human housekeeping genes, and the like; the pathogens include, but are not limited to, viruses, bacteria, fungi, and the like.

In some embodiments, the reverse primer comprises a universal tag sequence and contains a phosphorylation modification group at the 5' end. In this embodiment, during the symmetric PCR amplification process, a sequence complementary to the universal tag sequence is replicated at the 3' end of the sense strand, and said complementary sequence guides the specific binding of the sense strand by the universal detection probe. The forward primer is an unmodified primer.

In some embodiments, a molar ratio of the forward primer to the reverse primer is 1:1.

In some embodiments, the exonuclease is a Mg²⁺-dependent 5'→3' exonuclease. The exonuclease selectively recognizes and degrades the DNA strand with 5' phosphorylation modification (in some embodiments, the strand extended from the reverse primer), thereby retaining the unmodified complementary strand (in some embodiments, the strand extended from the forward primer) to generate a high-purity single-stranded DNA (ssDNA) product retaining the sequence complementary to the universal tag sequence.

In some embodiments, the exonuclease is Lambda exonuclease and/or T7 exonuclease.

In some embodiments, the kit further comprises a signal amplification system capable of cooperating with the signal label to amplify a detection signal.

In some embodiments, the signal label is a gold nanoparticle label, and the kit further comprises a silver staining reagent.

In some embodiments, the kit further comprises a high-fidelity DNA polymerase and an exonuclease buffer.

Further, the exonuclease buffer comprises Mg²⁺, specifically comprising 2 mM MgCl₂.

Further, the exonuclease buffer comprises 10 mM Tris-HCl (pH 8.0), 2 mM MgCl₂, 50 mM KCl, and 0.1% DMSO.

In some embodiments, the kit further comprises dNTPs and an amplification buffer.

In some embodiments, the solid support is a gene chip, a microfluidic chip, a glass slide, a nylon membrane, a nitrocellulose membrane, a magnetic bead, or a paper-based chip.

Further, the solid support is a gene chip. Further, the solid support is an aldehyde-functionalized glass slide or an amino-functionalized silicon substrate.

In some embodiments, the capture probe is immobilized on the surface of the solid support via a covalent bond or physical adsorption.

A second aspect of the present disclosure provides a primer composition as described in any one of the above.

A third aspect of the present disclosure provides a high-sensitivity and high-specificity nucleic acid detection method based on symmetric barcode PCR and Lambda exonuclease.

The nucleic acid detection method provided by the present disclosure comprises the following steps:
(1) performing symmetric PCR amplification on a target nucleic acid to be tested using a forward primer and a reverse primer to obtain a double-stranded DNA; wherein one of the forward primer and the reverse primer comprises a universal tag sequence and contains a phosphorylation modification group at the 5' end;
(2) reacting the double-stranded DNA obtained in step (1) with an exonuclease to degrade the strand containing the 5' phosphorylation modification, so as to obtain a single-stranded DNA;
(3) performing hybridization and detection on the single-stranded DNA obtained in step (2) using a capture probe and a universal detection probe; wherein the universal detection probe comprises a universal tag sequence capable of hybridizing with the single-stranded DNA and a signal label for signal recognition.

In some embodiments, in step (3), a specific sequence region in the single-stranded DNA hybridizes with the capture probe, and simultaneously or subsequently, a sequence in the single-stranded DNA complementary to the universal tag sequence hybridizes with the universal detection probe.

In some embodiments, in step (3), the signal label is a gold nanoparticle; after the hybridization is completed, a silver staining reagent is added, and qualitative or quantitative analysis is performed by detecting a grey value, an optical density value, or the number of silver particles of deposited spots.

In some embodiments, the reaction conditions for step (2) are: incubating at 30°C-40°C for 5-15 minutes; after the reaction in step (2) is completed, the method further comprises a step of inactivating the exonuclease or directly performing the hybridization reaction of step (3).

In some embodiments, the target nucleic acid to be tested is RNA or DNA; when the target nucleic acid to be tested is RNA, the detection method further comprises a step of using random primers for reverse transcription to produce a cDNA template.

In some embodiments, the nucleic acid detection method simultaneously detects a plurality of target nucleic acids to be tested.

Specifically, the forward primer and the reverse primer are multiple sets of specific primer pairs designed for a plurality of pathogen targets to be detected; the symmetric PCR amplification is multiplex PCR amplification performed on a test sample using the multiple sets of specific primer pairs to obtain double-stranded DNA for the plurality of pathogens at one time. Specific capture probes designed for the plurality of target nucleic acids to be tested are immobilized at different positions on the gene chip, and are capable of specifically capturing the single-stranded DNA formed after amplification and enzymatic digestion of the corresponding pathogens; the objective of detecting the pathogens to be tested is achieved through a universal sequence-labelled gold nanoparticle-silver staining process.

In some embodiments, the nucleic acid detection method utilizes the kit according to any one of the above for detection.

In some embodiments, the nucleic acid detection method is a nucleic acid detection method for non-diagnostic purposes.

In some embodiments, the nucleic acid detection method comprises the following steps:
Symmetric barcode PCR amplification: using a pair of primers to amplify a target nucleic acid, wherein the 5' end of the reverse primer contains a phosphorylation modification and introduces a universal tag sequence, to generate a double-stranded DNA amplicon comprising the universal sequence;

Lambda exonuclease digestion: reacting the amplification product with Lambda exonuclease to selectively degrade the 5'-phosphorylated strand, generating a high-purity single-stranded DNA product;

Chip hybridization detection: hybridizing the single-stranded DNA product with a capture probe on a gene chip, and achieving quantitative analysis of the target through the binding of a detection probe (containing a gold nanoparticle label) and silver staining development.

By applying the above technical solutions, the present disclosure has the following advantages over the prior art:
The kit and nucleic acid detection method of the present disclosure combine the high-efficiency amplification capability of symmetric PCR, the specific degradation characteristics of exonuclease for preparing high-purity single-stranded DNA carrying a sequence complementary to a universal tag, and the formation of a "capture probe-target ssDNA-universal detection probe" sandwich complex through hybridization, thereby improving detection sensitivity and specificity.

The present disclosure solves the long-standing technical problems of low sensitivity caused by dsDNA re-annealing in traditional symmetric PCR chip hybridization and the low amplification efficiency and complex optimization of asymmetric PCR. By integrating symmetric PCR with selective exonuclease digestion and a universal gold nanoparticle-silver staining detection system, the invention achieves ultra-high sensitivity (LOD reaching 10 copies/mL), excellent multiplex capability, and significantly reduced false-positive rates through triple specificity screening, providing a robust, cost-effective, and POCT-suitable solution that is markedly superior to conventional fluorescence-based and hybridization methods.

### BRIEF DESCRIPTION OF THE DRAWINGS

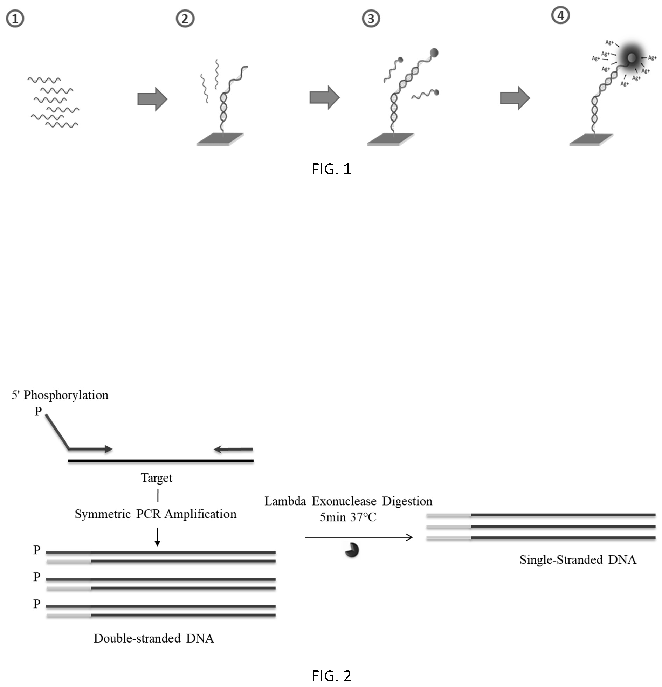

FIG. 1 is a schematic diagram illustrating the principle of chip hybridization reaction and gold nanoparticle-silver staining signal amplification according to one embodiment of the present disclosure, wherein:
   - ① represents a single-stranded DNA obtained after symmetric PCR amplification and exonuclease degradation;
   - ② represents the single-stranded DNA being captured by a specific probe on the chip;
   - ③ represents a probe coupled with gold nanoparticles specifically binding to the captured target to form a sandwich complex; and
   - ④ represents the addition of a silver staining enhancement solution, wherein the gold nanoparticles catalyze the deposition of silver particles to achieve signal amplification.
FIG. 2 is a flow chart illustrating the principle of preparing single-stranded DNA based on symmetric PCR amplification and enzymatic digestion according to the present invention.
FIG. 3 is a comparison of chip signal intensities of three different reaction systems (asymmetric PCR reaction, asymmetric PCR reaction plus enzymatic digestion, and symmetric PCR reaction plus enzymatic digestion) according to one embodiment of the present disclosure, wherein the target is an HPV3 gene.
FIG. 4 is a diagram showing the results of octaplex amplification and detection of seven clinical pathogen-related targets and one human internal control target according to one embodiment of the present disclosure.
FIG. 5 is a linear regression of silver count values measured for an E. coli strain in the present detection system according to one embodiment of the present disclosure.
FIG. 6 shows the detection results of three pathogens, ECLO, SAUR, and PMIR, at different concentrations according to one embodiment of the present disclosure.
FIG. 7 shows the detection results of two pathogens, E. coli and ABAU, at different concentrations according to one embodiment of the present disclosure.

### DETAILED DESCRIPTION OF THE INVENTION

To address the deficiencies of the prior art, the kit and nucleic acid detection method of the present disclosure utilize symmetric PCR amplification technology to efficiently amplify target nucleic acids, generating double-stranded DNA (dsDNA) comprising a universal tag sequence and a 5' phosphorylation modification on one strand. Subsequently, an exonuclease is used to digest the phosphorylated strand of the dsDNA to produce pure single-stranded DNA (ssDNA). These ssDNA molecules hybridize respectively with capture probes immobilized on a chip and detection probes labelled with gold nanoparticles. The signal intensity is then enhanced via silver staining imaging technology to achieve high-sensitivity and high-specificity nucleic acid detection. This method improves detection throughput and accuracy, and is particularly suitable for fields such as gene expression level detection, mutation analysis, pathogen identification, disease diagnosis, and drug screening in point-of-care testing (POCT) scenarios. Compared with traditional chip hybridization methods, the present disclosure significantly improves signal intensity and detection limits while reducing the false positive rate.

Specifically, the solution of the present disclosure offers the following advantages:
1. Ultra-high detection sensitivity: Experimental results demonstrate that in common clinical sample matrices (e.g., throat swabs, sputum reverse-transcription cDNA samples), the limit of detection (LOD) of this method can reach 10 copies/mL. This is approximately 10 times higher than that of qPCR detection reagents. This success relies on the thorough removal of the complementary strand by exonuclease digestion, which eliminates competitive re-annealing, while symmetric PCR amplification ensures high abundance of the initial product.
2. High amplification efficiency and simplified process: By employing a symmetric PCR amplification mode, the amplification efficiency is nearly 100%, exhibiting typical exponential growth. This eliminates the need for tedious optimization of primer ratios required by asymmetric PCR. The reaction system possesses strong robustness with no significant mutual interference between primers, making it particularly suitable for the construction of multiplex PCR amplification systems.
3. Strong versatility and cost reduction: The introduction of a "universal tag sequence" design allows the same gold nanoparticle-labelled universal detection probe to be used regardless of the target being detected. This removes the need to individually design and synthesize specific detection probes for different targets, greatly reducing reagent preparation costs and process complexity, which facilitates large-scale production.
4. High specificity and effective elimination of false positives: A valid detection signal is generated only when three serial conditions are simultaneously met: "target-dependent specific primer amplification," "sequence-specific binding of the solid-phase capture probe on the chip," and "specific pairing between the sequence complementary to the universal tag sequence and the universal tag sequence of the detection probe." This forms a triple-specificity screening mechanism that effectively avoids false-positive results caused by non-specific amplification or cross-hybridization.

It should be noted that, unless otherwise defined, technical or scientific terms used in the present disclosure shall have the ordinary meaning as understood by those skilled in the art to which the present disclosure belongs.

Unless otherwise specified, the experimental methods in the following embodiments are conventional methods. Unless otherwise specified, the raw materials, reagents, and other materials used in the following embodiments are commercially available products. For example, the enzyme used for multiplex PCR is the product with catalogue number CW3219 manufactured by CWBIO; the Lambda exonuclease used is the product with catalogue number D7084L manufactured by Beyotime. The hybridization buffer may be purchased from Invitrogen (Catalogue No. 15591043); the DNA-Au binding buffer may be purchased from Invitrogen (Catalogue No. AM9767); and the silver staining reagents A and B may be purchased from Bio-Rad (Catalogue No. 1610449).

Unless otherwise specified, "%" refers to a percentage by mass (w/w).

The number of silver particles can be counted using a microscopic image analysis system or the like.

As used in this specification, "optional" or "optionally" means that the subsequently described event or circumstance may or may not occur, and that the description includes instances where the event or circumstance occurs and instances where it does not.

The technical solutions provided by the present disclosure are further described below in conjunction with specific embodiments. The following embodiments are only intended to illustrate the present disclosure and are not intended to limit the scope of protection of the present disclosure.

### Embodiment 1: Principle of Nucleic Acid Detection Based on Symmetric Barcode PCR and Exonuclease-Assisted Digestion

This embodiment illustrates the core mechanism of the method, as shown in FIG. 1 and FIG. 2.

### 1. Primer Strategy:

Reverse Primer (RP): Designed as "5'-phosphate group (5'-P) + universal tag sequence (Universal Tag) + specific reverse sequence." That is, all functional modifications (phosphorylation and the Tag) are concentrated on the reverse primer.

Forward Primer (FP): Designed as a "specific forward sequence" with a 5'-OH (conventional unmodified).

2. Amplification and Replication: Conventional symmetric PCR is performed.

During the amplification process, the reverse primer extends to form an antisense strand, the 5' end of which carries the phosphate group and the universal tag sequence.

Crucially, when DNA polymerase extends the forward primer using the antisense strand as a template, it replicates the complementary sequence of the universal tag (Complementary Tag) onto the 3' end of the newly generated forward strand (sense strand).

The final product is a double-stranded DNA: an antisense strand (5'-P, 5'-Tag) and a forward strand (5'-OH, 3'-Complementary Tag).

3. Enzymatic Digestion: Lambda exonuclease is added. This enzyme is a highly selective 5'→3' exonuclease that specifically recognizes and degrades DNA strands carrying a 5'-phosphate group.

The result is that the antisense strand carrying the 5'-P is step-wise degraded into mononucleotides, while the forward strand is completely retained because the 5'-OH cannot be recognized by the enzyme.

4. Hybridization: The retained high-purity single-stranded DNA (i.e., the forward strand) possesses dual binding functions:

Its middle region contains a target-specific sequence used for complementary binding with a solid-phase capture probe on the chip;
Its 3' end contains the complementary sequence of the universal tag (i.e., a universal binding region) used for specific binding with a universal detection probe.

5. Color Development: The capture probe on the chip grabs the middle region of the ssDNA to immobilize the target; the gold nanoparticle-labelled universal detection probe grabs the universal binding region at the 3' end of the ssDNA. The three components form a stable "sandwich" hybridization structure. Subsequently, a silver staining reagent is added, and silver ions are catalytically reduced and deposited on the surface of the gold nanoparticles, forming black spots visible to the naked eye.

### Embodiment 2: High-Sensitivity Detection of HPIV3 Virus (Higher Efficiency in Obtaining Target Single Strands than Asymmetric Amplification)

### 1. Main Reagent Formulation:

In this example, the formulation of the Lambda exonuclease buffer (1×) used is as follows:
10 mM Tris-HCl (pH 8.0)
2 mM MgCl₂
50 mM KCl
0.1% DMSO

### 2. Material Preparation:

Primers were designed targeting the conserved region of Human Parainfluenza Virus Type 3 (HPIV3).
Forward Primer (FP): 5'-TCTTGGGCCTGTGTTATCTC-3' (SEQ ID NO: 1).
Reverse Primer (RP): 5'- (Phosphate) - [Universal Tag A] -AGGGTGGCAAGTGGCTCC-3' (SEQ ID NO: 2).
Note: The 5' end of the RP is modified with a phosphate group; the sequence of Universal Tag A is: 5'-TCT CAA CTC GTA GCT-3' (SEQ ID NO: 3).
Chip Capture Probe: Designed for the HPIV3 specific fragment with the sequence: 5'-TCTTCTGGTCAGAGACAGTAATAGACGA-3' (SEQ ID NO: 4), immobilized on an aldehyde-functionalized silicon substrate surface.
Universal Detection Probe: Gold nanoparticles modified with an oligonucleotide identical to Universal Tag A.

### 3. Symmetric PCR Amplification:

A 25 µL reaction system was constructed:
5× PCR Master Mix: 5 µL
FP (10 µM): 0.2 µL
RP (10 µM): 0.2 µL (Molar ratio of FP to RP is 1:1)
cDNA template from reverse-transcription of HPIV3 RNA: 2 µL
ddH₂O: up to 25 µL
Reaction Procedure: 95°C for 3 min; (95°C for 10 s, 60°C for 30 s) × 35 cycles; 72°C for 5 min.

### 4. Lambda Exonuclease Digestion:

Take 18 µL of the above PCR product.

Add 1 µL of Lambda Exonuclease (5 U/µL) and 2 µL of 10× digestion buffer.

Reaction Conditions: Incubate at a constant temperature of 37°C for 5-15 minutes. During this step, the reverse strand carrying the 5' phosphate group is degraded by the enzyme. Termination: The ambient temperature (60°C for 10 minutes) used for subsequent hybridization of the amplification product with the silicon chip capture probe is sufficient to inactivate the enzyme. At this point, the system primarily contains forward single-stranded DNA comprising the sequence complementary to Universal Tag A.

### 5. Chip Hybridization and Color Development:

Mix the digested product with hybridization buffer, load onto the gene chip, and hybridize at 60°C for 10 minutes.

Add the gold nanoparticle-labelled universal detection probe to the hybridization system and continue incubating at 60°C for 20 minutes.

After washing, add the prepared silver staining solution and react at 42°C for 3 minutes in the dark.

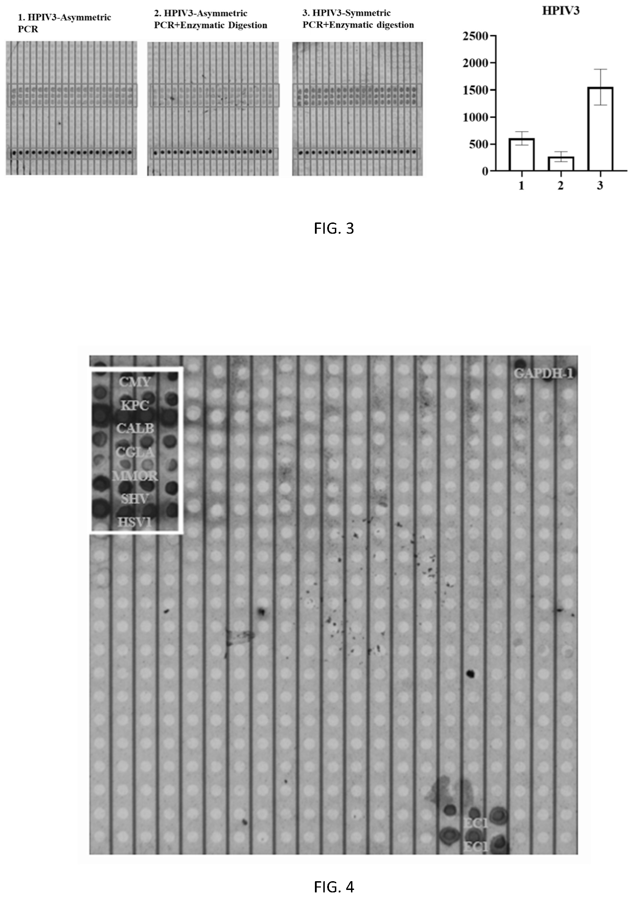

Results: As shown in FIG. 3, clear black spots appeared at the corresponding sites on the chip. Based on greyscale analysis, the signal intensity of Experimental Group 3 (Symmetric PCR + Digestion) was significantly superior to those of Experimental Group 1 (Asymmetric PCR) and Experimental Group 2 (Asymmetric PCR + Digestion). Statistical analysis of spot data shows that the chip signal of Experimental Group 3 is approximately three times that of Experimental Group 1.

### Embodiment 3: Implementation of a Multiplex Pathogen Detection Method

### 1. Main Reagent Formulation:

In this example, the formulation of the Lambda exonuclease buffer (1×) used is as follows: 10 mM Tris-HCl (pH 8.0)
2 mM MgCl₂
50 mM KCl
0.1% DMSO

### 2. Material Preparation:

Primers were designed targeting conserved regions of specific gene loci for common pneumonia pathogens, drug-resistance gene targets, and human gene targets. Detailed information regarding the primers and probes is provided in Table 1 below.

**Table 1. Primer, Reverse Primer, and Capture Probe Sequences Used in the Multiplex Pathogen Detection Assay (Embodiment 3)**

| **Gene Information** | **Forward Primer (5'→3')** | **Reverse Primer (5'→3')** | **Capture Probe (5'→3')** |
|---|---|---|---|
| CMY (CP092596.1) | CCCTATTATTTCACCT GGGGT (SEQ ID NO:5) | CGTATAGGTGGCTAA GTGC (SEQ ID NO:6) | CACGCCGTTAAACGTCTT ACTAACCGA (SEQ ID NO:7) |
| KPC (MN433457.1) | TATTATTTCACCTGG GGTAA (SEQ ID NO:8) | CGTATAGGTGGCTAA GTGCA (SEQ ID NO:9) | CGGCCCGGCCGGGCTGA CGGCCTTC (SEQ ID NO:10) |
| CALB (GU319992.1) | CGGGTCTGTGTAAA GTTCCTTCG (SEQ ID NO:11) | AATCCCGTGCGATGA GATG (SEQ ID NO:12) | CAGCATCGGTTTGGAGC GGCAGGATAATGG (SEQ ID NO:13) |
| CGLA (KY106474.1) | ACAGGACGTCATGG AGGGT (SEQ ID NO:14) | TCGGTCCCGGCTGGC CGTA (SEQ ID NO:15) | TGTCAGTTCTTTGTAAAG GGTGCTCGAA (SEQ ID NO:16) |
| MMOR (KP069480.1) | TGCCAGGGTGCGGA TATTTT (SEQ ID NO:17) | ACGCATTGCTGATCG ACTG (SEQ ID NO:18) | GGGCTCATGAGCTGGAT GCGGT (SEQ ID NO:19) |
| SHV (JX121120.1) | CCGCGTAGGCATGA TAGAAAT (SEQ ID NO:20) | GCTGGCGATAGTGGA TCTTT (SEQ ID NO:21) | GTGCTGGCGCGGGTGG ATGCCGGT (SEQ ID NO:22) |
| HSV1 (KY274362.1) | GGCCCAACATATCGT TGACAT (SEQ ID NO:23) | CTGTACGTGCGGGAA CAC (SEQ ID NO:24) | TTTACGTACAACCACATA CAGCGCCATGT (SEQ ID NO:25) |
| GAPDH (NM_00135794 3.2) | ACCACAGTCCATGCC ATCAC (SEQ ID NO:26) | CCAGTGAGCTTCCCG TTCAG (SEQ ID NO:27) | GTGGTGAAGCAGGCGTC GGAGGGCCCCT (SEQ ID NO:28) |

| | | | |
|---|---|---|---|
| **Note:** All reverse primers contain a 5'-phosphate modification and the universal tag sequence 5'-TCT CAA CTC GTA GCT-3' (SEQ ID NO: 3). Data are representative of triplicate experiments. Chip Capture Probes: Chip capture probes designed for the characteristic gene fragments of the aforementioned pathogens are immobilized on an aldehyde-functionalized silicon substrate surface. | | | |

Universal Detection Probe: Gold nanoparticles modified with an oligonucleotide identical to Universal Tag A.

### 3. Symmetric PCR Amplification

A 25 µL reaction system was constructed:
5× PCR Master Mix: 5 µL
FP (10 µM): 0.2 µL
RP (10 µM): 0.2 µL (Molar ratio of FP to RP is 1:1)
DNA template or cDNA template from reverse-transcription of RNA: 2 µL
ddH₂O: up to 25 µL
Reaction Procedure: 95°C for 3 min; (95°C for 10 s, 60°C for 30 s) × 35 cycles; 72°C for 5 min. The FPs for the characteristic genes of the pathogens and the human gene were mixed at an equimolar ratio, and the RPs for the characteristic genes of the pathogens and the human gene were mixed at an equimolar ratio.

### 4. Lambda Exonuclease Digestion

Take 18 µL of the above PCR product.

Add 1 µL of Lambda Exonuclease (5 U/µL) and 2 µL of 10× digestion buffer.

Reaction Conditions: Incubate at a constant temperature of 37°C for 5-15 minutes. During this step, the reverse strand carrying the 5' phosphate group is degraded by the enzyme. Termination: The ambient temperature (60°C for 10 minutes) used for subsequent hybridization of the amplification product with the silicon chip capture probe is sufficient to inactivate the enzyme. At this point, the system primarily contains forward single-stranded DNA comprising the sequence complementary to Universal Tag A.

### 5. Chip Hybridization and Color Development

Mix the digested product with hybridization buffer, load onto the gene chip, and hybridize at 60°C for 10 minutes.

Add the gold nanoparticle-labelled universal detection probe to the hybridization system and continue incubating at 60°C for 20 minutes.

After washing, add the prepared silver staining solution and react at 42°C for 3 minutes in the dark.

Results: As shown in FIG. 4, seven different targets in the multiplex detection showed positive results with uniform signal intensity, and no false-positive signals were generated at other positions on the chip (where capture probes for other pathogens were immobilized). This indicates that the system has achieved simultaneous detection of multiplex pathogens with high uniformity.

As further shown in FIG. 4, the GAPDH gene was amplified normally. Since human samples contain the human genome and the expression level of the selected housekeeping gene is essentially constant, the intensity of the human internal control gene in a fixed volume of sample can be used to calibrate the copy number of pathogens in the sample, thereby assisting in the quantitative analysis of the pathogen detection targets.

### Embodiment 4: Implementation of a Quantitative Multiplex Pathogen Detection Method

This section demonstrates the technical details of the present method for achieving quantitative multiplex pathogen detection using five common clinical pathogenic bacteria as examples. The five pathogens include Escherichia coli (ATCC8739, abbreviated as Ecoli), Acinetobacter baumannii (ATCC19606, abbreviated as ABAU), Staphylococcus aureus (ATCC12600, abbreviated as SAUR), Proteus mirabilis (ATCC29906, abbreviated as PMIR), and Enterobacter cloacae (ATCC13047, abbreviated as ECLO).

### 1. Main Reagent Formulation:

In this example, the formulation of the Lambda exonuclease buffer (1×) used is as follows: 10 mM Tris-HCl (pH 8.0)
2 mM MgCl₂
50 mM KCl
0.1% DMSO

### 2. Material Preparation:

Primers were designed targeting conserved regions of specific gene loci for these five pathogens. Detailed information regarding the primers and probes is provided in Table 2 below.

**Table 2. Primer and Capture Probe Sequences for Quantitative Multiplex Detection of Five Pathogenic Bacteria (Embodiment 4)**

| **Gene Information** | **Forward Primer (5'→3')** | **Reverse Primer (5'→3')** | **Capture Probe (5'→3')** |
|---|---|---|---|
| Ecoli (AP026110.1) | ACACAAGGGGATAC ACTTGCG (SEQ ID NO:29) | CCCATTTCTCCAGTG AAGATGGC (SEQ ID NO:30) | ATATCCGTTTATTGAACTG TTGATTA (SEQ ID NO:31) |
| ABAU (CP091334.1) | CAACTTGGGATGCTG TTCGCC (SEQ ID NO:32) | CGACCTTGCCGATCA ACACG (SEQ ID NO:33) | CGTTATAATTTTCTTGTAA AGC (SEQ ID NO:34) |
| SAUR (CP020020.1) | CATAAGCAACTTTAG CCAAGCC (SEQ ID NO:35) | AGCGATTGATGGTG ATACGG (SEQ ID NO:36) | ACAAAGCATCCTAAAAAA GGTGTAGAGA (SEQ ID NO:37) |
| ECLO (CP092042.1) | ATGCATTCGGTGGTG ATCATCAG (SEQ ID NO:38) | GGTAGAAGAAGGCG TGGTTGC (SEQ ID NO:39) | ACTGAAGAATACGGCAAC ATGATCGACTTC (SEQ ID NO:40) |
| PMIR (Z18752.1) | GCGGTAGACATTGCT GAAGTAACG (SEQ ID NO:41) | TGGGCAAACTATCAC AGTCACC (SEQ ID NO:42) | GAAACTGAATTACAATCT GTGTTCTC (SEQ ID NO:43) |

| | | | |
|---|---|---|---|
| **Note:** All reverse primers contain a 5'-phosphate modification and the universal tag sequence 5'-TCT CAA CTC GTA GCT-3' (SEQ ID NO: 3). Data are representative of triplicate experiments. | | | |

Chip Capture Probes: Chip capture probes designed for the characteristic gene fragments of the aforementioned pathogenic bacteria are immobilized on an aldehyde-functionalized silicon substrate surface.

Universal Detection Probe: Gold nanoparticles modified with an oligonucleotide identical to Universal Tag A on their surfaces.

### 3. Symmetric PCR Amplification

A 25 µL reaction system was constructed:
5× PCR Master Mix: 5 µL
FP (10 µM): 0.2 µL
RP (10 µM): 0.2 µL (Molar ratio of FP to RP is 1:1)
DNA template or cDNA template from reverse-transcription of RNA: 2 µL
ddH₂O: up to 25 µL

Reaction Procedure: 95°C for 3 min; (95°C for 10 s, 60°C for 30 s) × 35 cycles; 72°C for 5 min. The FPs for the characteristic genes of the aforementioned pathogenic bacteria were mixed at an equimolar ratio, and the RPs for the characteristic genes of the aforementioned pathogenic bacteria were mixed at an equimolar ratio.

### 4. Lambda Exonuclease Digestion

Take 18 µL of the above PCR product.

Add 1 µL of Lambda Exonuclease (5 U/µL) and 2 µL of 10× digestion buffer.

Reaction Conditions: Incubate at a constant temperature of 37°C for 5-15 minutes. During this step, the reverse strand carrying the 5' phosphate group is degraded by the enzyme. Termination: The ambient temperature (60°C for 10 minutes) used for subsequent hybridization of the amplification product with the silicon chip capture probe is sufficient to inactivate the enzyme. At this point, the system primarily contains forward single-stranded DNA comprising the sequence complementary to Universal Tag A.

### 5. Chip Hybridization and Color Development

Mix the digested product with hybridization buffer, load onto the gene chip, and hybridize at 60°C for 10 minutes.

Add the gold nanoparticle-labelled universal detection probe to the hybridization system and continue incubating at 60°C for 20 minutes.

After washing, add the prepared silver staining solution and react at 42°C for 3 minutes in the dark.

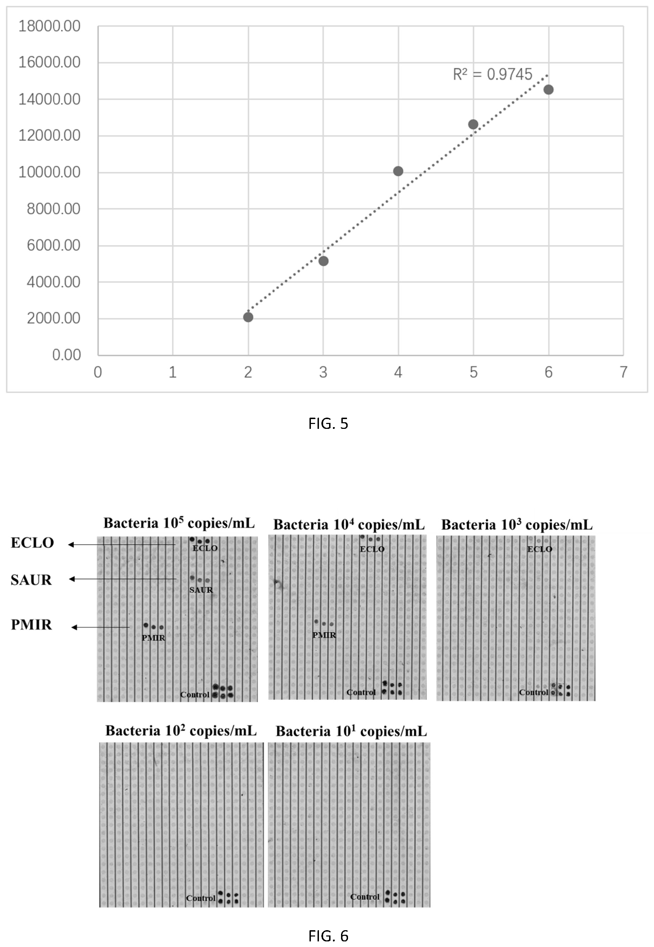

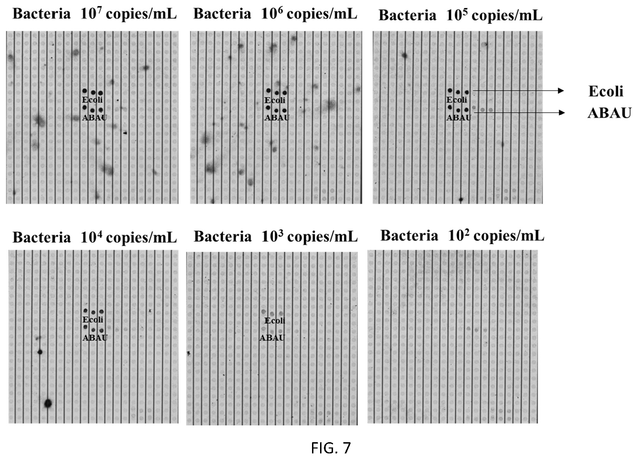

Results: As shown in Table 3, the number of silver particles observed in the detection of the five bacteria at different concentrations exhibits a partial linear relationship. As shown in FIG. 5 to FIG. 7, the linear range for the quantitative detection capability of each strain was determined based on the detected number of silver particles. Therefore, the multiplex detection system successfully achieved quantitative detection of different pathogenic bacterial targets.

**Table 3. Silver Particle Counts at Different Template Concentrations for Quantitative Multiplex Pathogen Detection (Embodiment 4)**

| **Tested Targets** | **Concentration (copies/ml)** | | | | | | |
|---|---|---|---|---|---|---|---|
| | **10⁷** | **10⁶** | **10⁵** | **10⁴** | **10³** | **10²** | **10¹** |
| Ecoli | 16802 | 14447 | 12690 | 10673 | 5074 | 2023 | 1192 |
| ABAU | 10253 | 12938 | 12863 | 9773 | 4770 | 2103 | 1048 |
| PMIR | 12304 | 11940 | 11335 | 6027 | 1176 | 988 | 895 |
| ECLO | 17489 | 16490 | 16986 | 8799 | 2747 | 1283 | 1135 |
| SAUR | 10934 | 7980 | 7851 | 1286 | 969 | 869 | 1077 |

| | | | | | | | |
|---|---|---|---|---|---|---|---|
| **Note:** Silver particle counts were determined by microscopic image analysis after silver staining. Data represent mean values from triplicate experiments (n=3). Linear regression analysis (FIG. 5-7) confirms quantitative capability within the tested range. | | | | | | | |

In this context, 10¹ - 10⁷ represent the orders of magnitude for the molecular copy numbers of the DNA template or the cDNA template derived from reverse-transcription of RNA in the PCR amplification reaction system, ranging from approximately 10¹ to 10⁷.

### Embodiment 5: Achieving Ultra-High Detection Sensitivity Superior to Traditional Fluorescence Detection Methods

This section uses three common clinical pathogenic bacteria as subjects to compare the detection sensitivity between the multiplex pathogen detection method of the present disclosure and the fluorescence detection products of leading brands. The three pathogens include Escherichia coli (ATCC8739, abbreviated as Ecoli), Staphylococcus aureus (ATCC12600, abbreviated as SAUR), and Candida glabrata (ATCC90030, abbreviated as CGLA)

### 1. Main Reagent Formulation:

In this example, the formulation of the Lambda exonuclease buffer (1×) used is as follows: 10 mM Tris-HCl (pH 8.0)
2 mM MgCl₂
50 mM KCI
0.1% DMSO

### 2. Material Preparation:

Primers were designed targeting conserved regions of gene loci for the three pathogenic bacteria. Detailed information regarding the primers and probes is provided in Table 4 below.

**Table 4. Primer and Capture Probe Sequences Used in the Sensitivity Comparison Assay (Embodiment 5)**

| **Gene Name** | **Forward Primer (5'→3')** | **Reverse Primer (5'→3')** | **Capture Probe (5'→3')** |
|---|---|---|---|
| Ecoli | ACACAAGGGGATACAC | CCCATTTCTCCAGTGAA | ATATCCGTTTATTGAACTG |
| (AP02611 0.1) | TTGCG (SEQ ID NO:29) | GATGGC (SEQ ID NO:30) | TTGATTA (SEQ ID NO:31) |
| SAUR (CP02002 0.1) | CATAAGCAACTTTAGC CAAGCC (SEQ ID NO:35) | AGCGATTGATGGTGAT ACGG (SEQ ID NO:36) | ACAAAGCATCCTAAAAAA GGTGTAGAGA (SEQ ID NO:37) |
| CGLA (KY106474 .1) | ACAGGACGTCATGGAG GGT (SEQ ID NO:14) | TCGGTCCCGGCTGGCC GTA (SEQ ID NO:15) | TGTCAGTTCTTTGTAAAGG GTGCTCGAA (SEQ ID NO:16) |

| | | | |
|---|---|---|---|
| **Note:** All reverse primers contain a 5'-phosphate modification and the universal tag sequence 5'-TCT CAA CTC GTA GCT-3' (SEQ ID NO: 3). Data are representative of triplicate experiments. | | | |

Chip Capture Probes: Chip capture probes designed for the characteristic gene fragments of the aforementioned pathogenic bacteria are immobilized on an aldehyde-functionalized silicon substrate surface.

Universal Detection Probe: Gold nanoparticles modified with an oligonucleotide identical to Universal Tag A.

### 3. Symmetric PCR Amplification

A 25 µL reaction system was constructed:
5× PCR Master Mix: 5 µL
FP (10 µM): 0.2 µL
RP (10 µM): 0.2 µL (Molar ratio of FP to RP is 1:1)
DNA template or cDNA template from reverse-transcription of RNA: 2 µL
ddH₂O: up to 25 µL
Reaction Procedure: 95°C for 3 min; (95°C for 10 s, 60°C for 30 s) × 35 cycles; 72°C for 5 min. The FPs for the characteristic genes of the aforementioned pathogenic bacteria were mixed at an equimolar ratio, and the RPs for the characteristic genes of the aforementioned pathogenic bacteria were mixed at an equimolar ratio.

### 4. Lambda Exonuclease Digestion

Take 18 µL of the above PCR product.

Add 1 µL of Lambda Exonuclease (5 U/µL) and 2 µL of 10× digestion buffer.

Reaction Conditions: Incubate at a constant temperature of 37°C for 5-15 minutes. During this step, the reverse strand carrying the 5' phosphate group is degraded by the enzyme. Termination: The ambient temperature (60°C for 10 minutes) used for subsequent hybridization of the amplification product with the silicon chip capture probe is sufficient to inactivate the enzyme. At this point, the system primarily contains forward single-stranded DNA comprising the sequence complementary to Universal Tag A.

### 5. Chip Hybridization and Color Development

Mix the digested product with hybridization buffer, load onto the gene chip, and hybridize at 60°C for 10 minutes.

Add the gold nanoparticle-labelled universal detection probe to the hybridization system and continue incubating at 60°C for 20 minutes.

After washing, add the prepared silver staining solution and react at 42°C for 3 minutes in the dark.

Results: The results are shown in Tables 5-7, wherein Table 5 shows the detection results of the fluorescence detection product from a commercial Fluorescent Quantitative PCR Kit, Table 6 shows the detection results of the present embodiment, and Table 7 provides a comparison of detection sensitivity between the method of the present embodiment and mainstream fluorescence detection products. In these tables, "live bacteria" refers to measurements based on the count of viable bacteria, where the nucleic acid molecules are extracted after lysis and used as the DNA template in the PCR amplification system; "DNA" refers to measurements based on the amount of pathogenic DNA template present in the PCR amplification system.

**Table 5. CT Values of the Commercial Fluorescence qPCR Kit for Live Bacteria and Purified DNA Templates (Embodiment 5)**

| **Tested Targets** | | **Concentration (CFU or copies/mL)** | | | | | | |
|---|---|---|---|---|---|---|---|---|
| | | **10⁶** | **10⁵** | **10⁴** | **10³** | **10²** | **10¹** | **10⁰** |
| Bacteria (CFU) | Ecoli | 23.46 | 28.01 | 32.73 | 36.35 | >40 | >40 | >40 |
| | SAUR | 32.74 | 34.8* | 37.72* | >40 | >40 | >40 | >40 |
| | CGLA | 33.55 | 38.19 | >40 | >40 | >40 | >40 | >40 |
| DNA (copies/mL) | Ecoli | 17.32 | 20.51 | 23.68 | 27.76 | 31.32 | 35.44 | 38.39* |
| | SAUR | 21.08 | 25.74 | 29.51 | 33.00 | 36.96 | >40 | >40 |
| | CGLA | 16.93 | 20.59 | 24.53 | 28.22 | 31.75 | 36.24 | >40 |

| | | | | | | | | |
|---|---|---|---|---|---|---|---|---|
| **Note:** Detection performed according to the manufacturer's recommended protocol. "*" indicates values near the limit of detection. Data represent mean values from triplicate experiments. | | | | | | | | |

**Table 6. Detection Results of the Present Method for Live Bacteria and Purified DNA Templates (Embodiment 5)**

| **Tested Targets** | | **Concentration (CFU or copies/mL)** | | | | | | |
|---|---|---|---|---|---|---|---|---|
| | | **10⁶** | **10⁵** | **10⁴** | **10³** | **10²** | **10¹** | **10⁰** |
| Bacteria (CFU) | Ecoli | 12707 | 11869 | 9107 | 4589 | 1710 | 1406 | 1759 |
| | SAUR | 10359 | 8948 | 5219 | 1840 | 1304 | 1485 | 1327 |
| | CGLA | 13095 | 11294 | 8380 | 3057 | 2090 | 1920 | 1793 |
| DNA (copies/mL) | Ecoli | 15235 | 13478 | 11093 | 10945 | 8903 | 4433 | 1706 |
| | SAUR | 14038 | 14404 | 11095 | 8870 | 5058 | 1476 | 1529 |
| | CGLA | 16711 | 14960 | 11295 | 9947 | 4967 | 1896 | 1621 |

**Table 7. Sensitivity Comparison Between the Present Method and the Commercial Fluorescence qPCR Kit (Embodiment 5)**

| **Sensitivity** | | **Present Method** | **Commercial Fluorescence qPCR Kit** | **Fold change compared to fluorescent method** |
|---|---|---|---|---|
| Bacteria (CFU) | Ecoli | 10³ | 10⁴ | 10 |
| | SAUR | 10⁴ | 10⁶ | 100 |
| | CGLA | 10³ | 10⁶ | 1000 |
| DNA (copies/mL) | Ecoli | 10 | 10² | 10 |
| | SAUR | 10² | 10³ | 10 |
| | CGLA | 10² | 10² | Equal |

| | | | | |
|---|---|---|---|---|
| **Note:** Improvement factor calculated as the ratio of the commercial kit's limit of detection to that of present method. The judgment method for positive samples is samples whose signal is greater than the average signal of negative control group samples plus 3 times the standard deviation. The method for judging detection sensitivity in this table is the minimum detection concentration at which positive sample signals appear in all three independent experiments. Data represent mean values from triplicate experiments. | | | | |

As shown by the experimental results, compared with the detection sensitivity of mainstream brand fluorescence detection products, the method of the present invention exhibits significant advantages in the detection of various types of pathogens and DNA.

The above embodiments are only intended to illustrate the technical concepts and features of the present disclosure, with the purpose of enabling those skilled in the art to understand the content of the present disclosure and implement it accordingly. They are not intended to limit the scope of protection of the present disclosure. Any equivalent changes or modifications made according to the spiritual essence of the present disclosure shall fall within the scope of protection of the present disclosure.

## Claims

1. A kit for high-sensitivity nucleic acid detection using symmetric barcoded PCR amplification and Lambda exonuclease, comprising: a primer composition configured to perform symmetric PCR amplification on a target nucleic acid to obtain double-stranded DNA, wherein the primer composition comprises a forward primer and a reverse primer designed for the target nucleic acid, and one of the forward primer and the reverse primer comprises a universal tag sequence and a phosphorylation modification at the 5' end; an exonuclease configured to degrade the strand containing the 5' phosphorylation modification in the double-stranded DNA to obtain single-stranded DNA; a solid support having a capture probe immobilized thereon, the capture probe being capable of hybridizing with the single-stranded DNA to bind the single-stranded DNA to the solid support; and a universal detection probe comprising a universal tag sequence capable of hybridizing with the single-stranded DNA and a signal label for signal recognition.

2. The kit of claim 1, wherein the universal tag sequence is located between the 5' phosphorylation modification and a target-specific sequence, and wherein the 5' end of the other of the forward primer and the reverse primer is a hydroxyl group or another group that cannot be recognized by an exonuclease, and does not contain a universal tag sequence.

3. The kit of claim 1, wherein the universal tag sequence has a length of 10 to 30 nucleotides.

4. The kit of claim 1, wherein the primer composition comprises multiple sets of primer compositions for a plurality of target nucleic acids to be tested, the capture probe comprises a plurality of capture probes for the plurality of target nucleic acids to be tested, and the plurality of capture probes are respectively immobilized at different positions on the solid support; and wherein the reverse primer comprises the universal tag sequence and the phosphorylation modification at the 5' end.

5. The kit of claim 1, wherein the exonuclease is Lambda exonuclease, T7 exonuclease, or a combination thereof.

6. The kit of claim 1, wherein the signal label is a gold nanoparticle label, and the kit further comprises a silver staining reagent.

7. The kit of claim 1, further comprising a high-fidelity DNA polymerase, dNTPs, an amplification buffer, and an exonuclease buffer.

8. The kit of claim 1, wherein the solid support is a gene chip, a microfluidic chip, a glass slide, a nylon membrane, a nitrocellulose membrane, a magnetic bead, or a paper-based chip; and wherein the capture probe is immobilized on the surface of the solid support via a covalent bond or physical adsorption.

9. A nucleic acid detection method, comprising the following steps: (1) performing symmetric PCR amplification on a target nucleic acid to be tested using a forward primer and a reverse primer to obtain double-stranded DNA, wherein one of the forward primer and the reverse primer comprises a universal tag sequence and a phosphorylation modification at the 5' end; (2) reacting the double-stranded DNA obtained in step (1) with an exonuclease to degrade the 5' phosphorylation-modified strand to obtain single-stranded DNA; and (3) performing hybridization and detection on the single-stranded DNA obtained in step (2) using a capture probe and a universal detection probe, wherein the universal detection probe comprises a universal tag sequence capable of hybridizing with the single-stranded DNA and a signal label for signal recognition.

10. The nucleic acid detection method of claim 9, wherein in step (3), a specific sequence region in the single-stranded DNA hybridizes with the capture probe, and simultaneously or subsequently, a sequence in the single-stranded DNA complementary to the universal tag sequence hybridizes with the universal detection probe.

11. The nucleic acid detection method of claim 9, wherein in step (3), the signal label is a gold nanoparticle, and after the hybridization is completed, a silver staining reagent is added to perform qualitative or quantitative analysis by detecting a grey value, an optical density value, or the number of silver particles of deposited spots.

12. The nucleic acid detection method of claim 9, wherein reaction conditions for step (2) comprise incubating at 30°C-40°C for 5-15 minutes; and after the reaction in step (2) is completed, the method further comprises a step of inactivating the exonuclease or directly performing the hybridization reaction of step (3).

13. The nucleic acid detection method of claim 9, wherein the target nucleic acid to be tested is RNA or DNA; and when the target nucleic acid to be tested is RNA, the method further comprises a step of using random primers for reverse transcription to produce a cDNA template prior to step (1).

14. The nucleic acid detection method of claim 9, wherein the method simultaneously detects multiple target nucleic acids to be tested.

15. The nucleic acid detection method of claim 9, wherein the method utilizes the kit according to claim 1 for detection.
